# EUROPEAN PATENT SPECIFICATION

(11) **EP 2 548 589 B1**
(45) Date of publication and mention of the grant of the patent: **06.08.2014**
(21) Application number: 11305935.6
(22) Date of filing: 18.07.2011
(51) Int. Cl.: A61M 1/00

(54) **Negative pressure wound treatment assembly**
Negativdruck-Wundbehandlungsanordnung
Ensemble de traitement de plaies à pression négative

(43) Date of publication of application: 23.01.2013
(73) Proprietor: LABORATOIRES URGO, 21300 Chenove (FR); Société de Developpement et de Recherche Industrielle, 21300 Chenove (FR)
(72) Inventor: De Samber, Marc, 3920 LOMMEL (BE); Weekamp, Johannes, 5741 DH BEEK EN DONK (NL); Jacobs, Bas, 5825 CS OVERLOON (NL); Aalders, Arnold, 5146 AH WAALWIJK (NL); Steinbrunn, Julien, 21000 DIJON (FR)
(74) Representative: Cabinet Plasseraud

(56) References cited:
- WO-A2-2010/006182
- FR-A- 1 163 907
- GB-A- 2 223 409
- US-A- 4 331 147
- US-A- 4 341 212
- US-A1- 2005 267 424

## Description

The present invention relates to a negative pressure wound treatment assembly.

Negative pressure wound treatment assemblies are generally used to treat open wounds on a patient's body by applying a controlled reduced pressure or vacuum on the wound in order to promote cell migration and possibly exudates evacuation, so as to help healing the wound. A negative pressure wound treatment assembly generally comprises a covering layer adapted to form an airtight barrier covering the wound and a vacuum system adapted to be connected to the covering layer to generate the reduced pressure on the wound when it is covered by the layer.

Known negative pressure wound treatment assemblies are either of an active type, in which the reduced pressure is created by a pump supplied with electrical power, or of a passive type, in which the reduced pressure is not created by a pump and does not require an electrical power supply. An example of a known negative pressure wound treatment assembly of the passive type is disclosed in US 2005/0267424.

Negative pressure wound treatment assemblies of the active type are generally used in a hospital environment. They are cumbersome and subjected to a high level of control as regards to safety requirements, for example to avoid any risk of haemorrhage.

Negative pressure wound treatment assemblies of the passive type are more adapted to home care. In particular, a vacuum system of these known passive assemblies implements a syringe mechanism which requires an actuation by a user of a moveable piston to enable the vacuum system to generate the reduced pressure. However, in addition to a complex structure, such vacuum system suffers from drawbacks in the generation of a controlled reduced pressure on the wound as required for negative pressure wound treatment, the reduced pressure being dependent of the actuation by the user. To overcome these drawbacks, another vacuum system of these known passive assemblies implements a compressed spring arranged to move the piston upon release. However, in such vacuum system, as the reduced pressure is generated, a reservoir filled with a retaining media collects the exudates. The reservoir can hardly be emptied so that the vacuum system, also of a complex structure, can only be used once.

The invention aims to solve the above mentioned problems.

The invention is defined by independent claim 1.

To this end, the invention provides a negative pressure wound treatment assembly comprising:
- a covering layer adapted to form an airtight barrier covering a wound, the covering layer presenting a bottom surface intended to face the wound,
- a vacuum system adapted to be connected to the covering layer to generate a reduced pressure on the wound when said covering layer covers said wound,
   wherein the vacuum system comprises:
- a chamber having a tightly closed space with a reduced pressure, said chamber comprising an end closed by a perforable cap,
- a connecting device adapted to be attached to the covering layer and to the chamber to form a communication passage between the bottom surface of the covering layer and the space of the chamber, the connecting device having a penetration member forming at least a part of the communication passage and having at least one needle portion adapted to perforate the cap of the chamber.

Hence, the invention provides a negative pressure wound treatment assembly of the passive type, suitable for home care and which is simple in structure and intuitive in use. The chamber has a predetermined reduced pressure, independent of a user actuation, and makes it possible to generate a controlled reduced pressure through its progressive pressurisation of the chamber. In addition, in the negative pressure wound treatment assembly of the invention, the chamber not only generates reduced pressure on the wound but can also collect exudates from the wound. The assembly can easily be used several times by changing the chamber. The assembly which prevents the use of an additional reservoir can also be made more compact.

In a particular embodiment, the connecting device comprises:
- a securing interface attached to the covering layer, said securing interface comprising a port opening in the bottom surface of the covering layer and forming a part of the communication passage,
- a holder having a housing adapted to receive the chamber, the holder comprising the penetration member arranged within the housing so as to perforate the cap of the chamber with the needle portion, the holder comprising a connector in communication with the needle portion of the penetration member and forming a part of the communication passage, the connector being adapted to be mounted on the securing interface in communication with the port.

The chamber can be any shape, spherical, rectangular or tubular. The holder will be adapted to the form of the chamber. In a preferred embodiment of the invention, the chamber is of tubular shape.

As regards to the holder, it may comprise a hollow cylindrical body, when the chamber is a tube, extending along an axis between opened proximal and distal ends and defining the housing, the penetration member being mounted within the body with the needle portion directed towards the proximal end. The distal end of the body may then be adapted to be removably secured to the securing interface.

Besides, the holder may comprise a plunger supporting the needle portion of the penetration member and the connector, said plunger being mounted within the housing so as to slide between a retracted position, in which the connector is fully contained in the housing, and an extended position, in which the connector at least partly protrudes outwardly from the housing. The plunger may be resiliently returned to the retracted position.

The connector may be adapted to be fitted within the port of the securing interface.

The connector may be a portion of the penetration member extending in a direction which is opposite to that of the needle portion.

As regards to the securing interface, it may comprise a cup having a concave inner surface defining a cavity intended to face the wound, the port opening within the cavity.

Besides, the securing interface may comprise a plug through which the port extends, the plug being adapted to be removably secured to the holder.

The connecting device may further comprise a flexible pipe removably secured to the securing interface and to the holder, said pipe delimiting a conduit forming a part of the communication passage.

To improve the modularity of the assembly by enabling some components on the assembly to be replaced or separated from each other, while other components remain attached to the body of the patient, the connecting device may further comprise at least one valve member moveable between a closing position, in which said valve member closes the communication passage, and an opening position, in which said valve member opens the communication passage.

In particular, the valve member may comprise a cover mounted on the needle portion of the penetration member, the cover being in the closing position in absence of external strain, said cover covering a tipped end of said needle portion in the closing position, the cover being adapted to be resiliently deformed to the opening position when an external strain is exerted so as to push the cover in a direction opposite to the tipped end, said cover being perforated by the tipped end in the opening position.

In the embodiment wherein the connecting device comprises a securing interface and a holder, the valve member may comprise a membrane on the securing interface, the membrane closing and opening the port respectively in the closing and opening positions.

In the embodiment wherein the connecting device further comprises a pipe, the valve member may comprise a membrane within the pipe, the membrane closing and opening the conduit respectively in the closing and opening positions.

Other objects and advantages of the invention will emerge from the following disclosure of particular embodiments of the invention given as non limitative examples, the disclosure being made in reference to the enclosed drawings in which:
- Figure 1 is a perspective view of a vacuum system according to a first embodiment of the invention, the vacuum system comprising a holder and a securing interface adapted to be directly secured to each other,
- Figure 2 is a longitudinal section view of the vacuum system of Figure 1,
- Figure 3 is a longitudinal section view of a reduced pressure wound therapy assembly implementing the vacuum system of Figure 1,
- Figure 4 is a longitudinal section view of a vacuum system according to a second embodiment of the invention, the holder and the securing interface being adapted to be secured to each other through a flexible pipe,
- Figure 5 is a longitudinal section view of a reduced pressure wound therapy assembly implementing the vacuum system of Figure 4.

On the Figures, the same reference numbers refer to the same or similar elements.

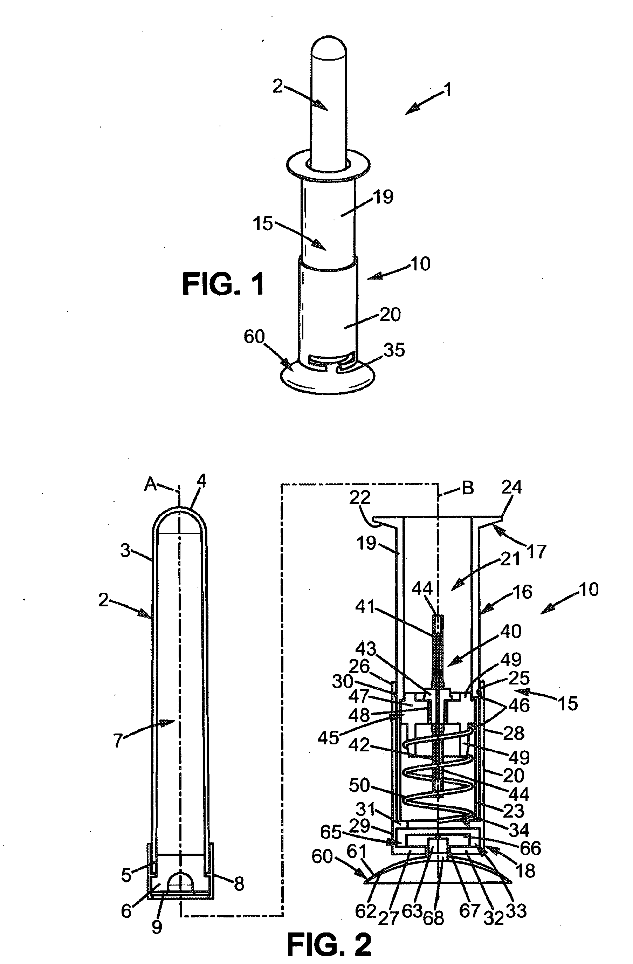

Figure 1 and 2 represent a first embodiment of a handheld passive vacuum system 1. As such, the vacuum system 1 forms a stand alone system able to generate a reduced pressure without the need of electrical power supply or of connection to any external power source.

On Figures 1 and 2, the vacuum system 1 comprises a chamber, in the form of an elongated tube 2 in the represented embodiment, and a connecting device 10.

The tube 2 comprises a cylindrical lateral wall 3 of circular cross section extending along an axis A from a closed round shaped end 4 to an opened end 5. A cap 6 made of perforable material, such as rubber or similar, is tightly fitted in the opened end 5 of the tube 2. The lateral wall 3 and the cap 6 of the tube 2 define a tightly closed space 7 pre-vacuumized at a reduced pressure, i.e. a pressure inferior to the atmospheric pressure. An outer ring 8 is fitted onto the cap 6 and a portion of the tube 2 surrounding the opened end 5 to seal the cap 6 to the tube 2. A sealing joint 9 may be interposed between the ring 8 and the cap 6. For example, the tube 2 can be of type of the tube exploited by the company GREINER BIO-ONE GmbH under the name VACUETTE ®.

As indicated, the space 7 of the tube 2 has a reduced pressure, below the atmospheric pressure. Depending on the application of the vacuum system 1, the tube 2 can be adapted to provide the required reduced pressure. Besides, as it will become apparent from the following, the vacuum system 1 may use one or several tubes 2, identical or different from each other, as a function of the reduced pressure to generate.

In the represented embodiment, the connecting device 10 comprises a holder 15 and a securing interface 60.

The holder 15 comprises a hollow cylindrical body 16 of circular cross section extending along an axis B between opened proximal 17 and distal 18 ends. In the represented embodiment, the body 16 is made of two parts, a sleeve 19 and a holder cap 20, for example of plastic material such as PMMA, assembled together.

The sleeve 19 is annular along an axis and has an inner surface defining a housing 21 adapted to coaxialy receive the tube 2. The sleeve 19 has a first end 22 defining the proximal end 17 of the body 16 and a second end 23 opposed to the first end 23. The first end 23 of the sleeve 19 is provided with a flange 24 flaring outwardly, substantially radially with respect to the axis of the sleeve 19. Besides, on an outer surface opposite to the inner surface, the sleeve 19 is provided with a peripheral outer rim 25.

The holder cap 20 extends along an axis between a first end 26 adapted to be connected to the sleeve 19 and a second end 27 forming the distal end 18 of the body 16. The holder cap 20 comprises an annular lateral wall 28 extending from the first end 26 along the axis, and a transverse end portion 29 perpendicular to the axis, arranged at the second end 27. The holder cap 20 has an inner surface adapted to be fitted onto the outer surface of the sleeve 19. Besides, to lock the holder cap 20 to the sleeve 19, the inner surface of the holder cap 20 is provided with a peripheral inner groove 30 adapted to receive the rim 25 of the sleeve 19.

The transverse end portion 29 of the holder cap 20 is configured to enable the body 16 to be removably attached to another element, as it will become apparent from the following of the description. In the illustrated embodiment, the transverse end portion 29 comprises two parallel inner 31 and outer walls 32 configured as a fork. The inner 31 and outer 32 walls define between them a groove 33 opened radially with respect to the axis of the holder cap 20. The inner wall 31 has a central hole 34 opening axially within the groove 33, and the outer wall 32 has a recess 35, visible on Figure 1, extending radially from a central portion to an edge, in correspondence with the groove 33 so as to define a border of a width narrower than that of the groove 33.

The holder 15 also comprises a penetration member 40 arranged within the housing 21 of the body 16. On Figures 1 and 2, the penetration member 40 is a dual needle assembly comprising two needle portions 41, 42 in communication and extending in opposite directions from a core 43. Each of the needle portions is formed of a sleeve with a through bore extending along an axis to a tipped end. In the represented embodiment, the needle portions 41, 42 are arranged coaxially, one 41 of the needle portions being directed towards the proximal end 17 of the body 16 so as to enable perforation of the cap 6 of the tube 2 by the tipped end, the other needle portion 42 acting as a connector so as to transfer the reduced pressure from the tube 2 to the tipped end of the connector 42. The core 43 of the penetration member 40 is provided with a securing element on an outer surface, such as an external thread.

Each of the needle portions 41, 42 of the penetration member 40 is covered by a cover 44, made of a resiliently deformable material such as rubber, forming a valve member. In absence of external strain, the cover 44 extends along the needle portion 41, 42 to an end that closes the tipped end of the needle portion 41, 42. In such rest state, the cover 44 is in a closing position, in which it impedes any flow of fluid through the needle portion 41, 42. When the cover 44 is pushed in a direction opposite to the tipped end of the needle portion 41, 42 by an external strain exerted on the end of the cover 44 towards the core 43 of the penetration member 40, the cover 44 is pierced by the tipped end of the needle portion 41, 42 and collapsed in the vicinity of the core 43 so as to uncover the needle portion 41, 42. In such strained state, the cover 44 is in an opening position, in which it allows the flow of fluid through the needle portion 41, 42. Upon release of the external strain, the cover 44 may resiliently return to the closing position.

In the embodiment represented on Figures 1 and 2, the penetration member 40 is mounted on a plunger 45.

The plunger 45 is cylindrical along an axis and presents a circular cross-section. The plunger 45 has an outer surface provided with opposite shoulders 46 extending radially with respect to the axis. In particular, the plunger 45 comprises a hub 47 provided with an axial central bore 48, and two opposite annular skirts 49 extending from the hub 47 along the axis. The outer surfaces of the skirts 49 are offset inwardly with respect to the outer surface of the hub 47 to form the shoulders 46. The central bore 48 has an inner surface provided with a securing element complementary to that of the penetration member 40, such as an internal thread, to enable a removable attachment of the penetration member 40 coaxially to the plunger 45.

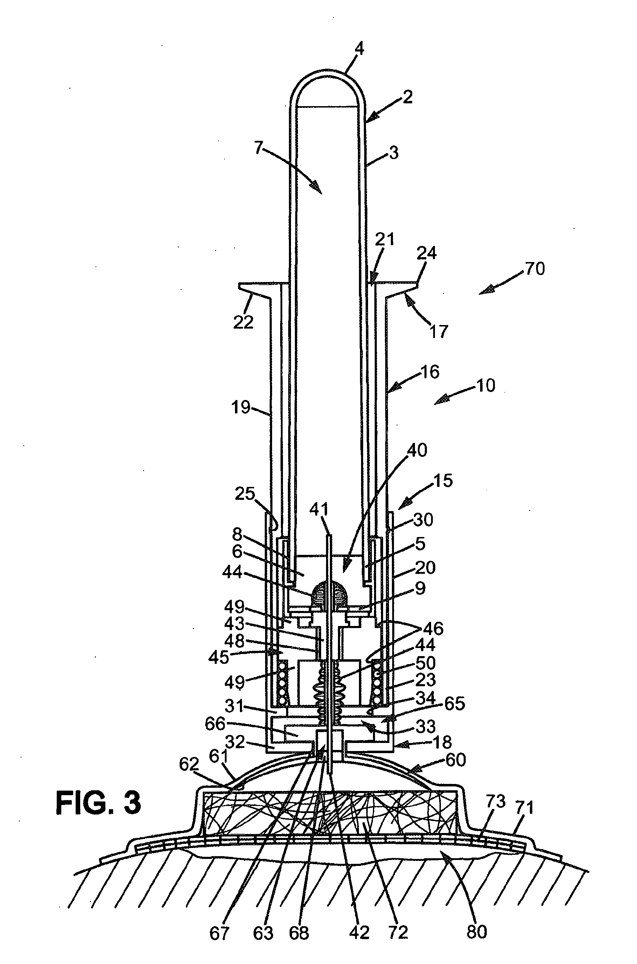

The plunger 45 is then coaxially mounted within the housing 21 of the body 16 with its outer surface in contact with the inner surface of the sleeve 19 so as to slide along the axis B of the body 16. As disclosed later on in relation to Figure 3, the plunger 45 can have a retracted position, in which the shoulder 46 directed towards the proximal end 17 of the body 16 abuts a corresponding shoulder on the inner surface of the sleeve 19. The shoulder of the sleeve 19 is arranged at a distance from the distal end 18 of the body so that the connector 42 is fully contained in the housing 21, without protruding from the distal end 18 of the body 16, in the retracted position of the plunger 45. The plunger 45 can move to an extended position, in which the skirt 49 directed towards the distal end 18 of the body 16 abuts the inner wall 31 so that the connector 42 at least partly protrudes outwardly from the housing 21 through the opening 34 and the recess 35 formed at the distal end 18 of the body 16.

In the represented embodiment, the plunger 45 is resiliently returned to the retracted position, for example by a spring 50 placed within the housing 21 and interposed between a seat formed on the inner wall 31 of the end portion 29 of the holder cap 20 around the opening 34, and another seat formed by the shoulder 46 of the plunger 45 facing this inner wall 31. In particular, the spring 50 is sized to make it possible to perforate the cap 6 of the tube 2 by the needle portion 41 while the plunger 45 remains in the retracted position and then to move the plunger 45 towards the extended position upon a further action on the tube 2 towards the distal end 18 of the holder 15 after the cap 6 has been perforated. A locking arrangement may be provided to maintain the plunger 45 in the extended position.

Turning now to the securing interface 60, it comprises a cup 61 formed of a curved wall extending transversally to a central axis to a circular outer edge. The wall has a concave inner surface 62 defining a cavity. A port 63 opening within the cavity extends centrally through the wall.

As can be seen on Figures 1 and 2, the distal end 18 of the body 16 and the securing interface 60 are adapted to be removably secured directly to one another.

To that end, the securing interface 60 also comprises a plug 65 secured to an outer surface of the cup 61 so as to be arranged along the central axis with the port 63 extending through the plug 65. The plug 65 is shaped and dimensioned to be removably secured directly to the distal end 18 of the holder 15. In particular, the plug 65 comprises a thick part 66 spaced apart from the cup 61 and connected to the cup 61 by a narrow part 67. The thick part 66 is adapted to be inserted transversally with respect to the axis B of the holder 15 in the groove 33 of the distal end 18 of the holder 15 while the narrow part 67 is slid along the recess 35 of the outer wall 32 of the distal end 18 of the holder 15. Once secured to the holder 15, the thick part 66 is received in the groove 33 of the distal end 18 of the holder 15, the narrow part 67 extending through the recess 35 of the outer wall 32 of the distal end 18 of the holder 15. The securing interface 60 is then coaxially mounted to the holder 15 with the port 63 arranged in correspondence with the connector 42 of the holder 15 so as to make it possible to mount the connector 42 on the securing interface 60 in communication with the port 63, and especially to fit the connector 42 within the port 63.

To enable a detachment of the holder 15 and the securing interface 60 without loosing vacuum within the cavity of the cup 61, a membrane 68 acting as a valve member is provided on the securing interface 60, for example in the plug 65, to selectively close and open the port 63 respectively in closing and opening positions.

Therefore, the needle portions 41, 42 of the holder 15 and the port 63 of the securing interface 60 form parts of a communication passage enabling the reduced pressure within the tube 2 to be applied to the inner surface 62 of the cup 61, within the cavity. The communication passage can be selectively opened and closed by the provision of the valve members 44, 68 to enable the tube 2, the holder 15 and the securing interface 60 to be separated from each other, especially for replacement or removal, while maintaining the reduced pressure within the cavity.

Figure 3 represents the vacuum system 1 of the first embodiment implemented in a reduced pressure wound treatment assembly 70 in which it is connected to a covering layer 71 to generate a reduced pressure on a wound 80 of a patient's body. Actually, it has been found that reduced pressure applied to the wound 80 improves the healing of the wound 80, especially by enhancing the migration of cells and by evacuating the exudates from the wound 80. In this respect, the vacuum system 1 of the invention takes advantage of the pre-vacuumized tube 2 that both generates the reduced pressure on the wound 80 in a controlled manner and collects the exudates.

In this application, the reduced pressure in the tube 2 is chosen to allow the suction of exudates at a determined rate for a determined duration. In a particular example, the rate is between 3 cc to 10 cc per hour, and preferably of about 5 cc per hour, and the duration is between several hours to several days, and preferably of about seventy-two hours. To apply the suitable reduced pressure for the desired duration, one tube 2 adapted accordingly or several identical or different tubes 2 can be implemented.

In such application, the vacuum system 1 offers an intuitive use for home care of the wound 80, especially by a nurse who is familiar with that kind of system analogous to that used for sampling blood.

The covering layer 71 can be any kind of element or combination of elements adapted to form an airtight barrier which makes it possible to obtain and maintain the vacuum applied by the vacuum system 1. This covering layer 71 is therefore impermeable to liquids and gases, thin and comfortable, and preferably transparent. Aptly, whilst the covering layer 71 is gas impermeable, the material of the covering layer 71 can have high moisture vapour permeability.

The covering layer 71 is made, for example, of a film that is generally adhering. The film can be, for example, a film made of polyurethane, polyether-polyester copolymer, polyester-polyamide copolymer, vinyl acetate ethylene copolymer or polyolefin. An adhesive makes it possible to attach the film to the skin of the patient at the periphery of the wound 80 so as to form the airtight barrier and to maintain the vacuum. This adhesive is chosen among the adhesives usually used for the manufacturing of dressings. For example, acrylic adhesives, hot melt adhesives, hydrocolloid adhesives or adhesives made of silicone or polyurethane can be used. As examples for suitable covering layers 71, the non absorbent films exploited by the company LABORATOIRES URGO under the name OPTISKIN® or by the company 3M under the name TEGADERM® can be cited.

In a variant, the covering layer 71 can be any king of material adapted to form an airtight barrier, such as, for example, the above mentioned films provided with an adhesive of not, and which is attached to a sealant that has previously been placed on the skin of the patient at the periphery of the wound 80. Such sealant is, for example, the product exploited by the company MOLNLYCKE HEALTH CARE under the name MEPISEAL®.

If necessary, the covering layer 71 can be combined with additional layers that will contact the wound 80 under the covering layer 71. In this respect, a spacing layer 72, which can be of any kind presenting a defined structure such as a honeycomb material, can be added to the covering layer 71. In a preferred embodiment of the invention, the spacing layer 72 is an absorbing material. For example, the covering layer 72 can be made of a textile material such as a non-woven material, an alveolar material such as an absorbing foam and, in particular, an hydrophilic polyurethane absorbing foam, or an assembly of these different absorbing materials. Such absorbing materials and their assembling are usually used in dressing manufacturing or in the hygiene field, for example in the manufacturing of diapers.

If necessary, in order to avoid the alteration of healing tissue by removal of the spacing layer 72, a wound contact layer 73 can be interposed between the latter and the wound 80. As wound contact layer 73, scaffolds made of materials biodegradable or not or the product exploited by the company LABORATOIRES URGO under the name URGOTUL ® can be used.

In a variant of the present invention, the above mentioned different layers and the covering layer 71 can be assembled to form a sole product and, in particular, an absorbent wound dressing. Examples of such absorbent wound dressings are exploited by LABORATOIRES URGO under the names URGOCELL® adhesive and URGOCELL® CONTACT adhesive, by the company MOLNLYCKE HEALTH CARE under the name MEPILEX® Border or by the company SMITH & NEPHEW under the name ALLEVYN^{™}.

If necessary, these absorbent wound dressings will be adapted by selecting the adhesives which make it possible to ensure the formation of the airtight barrier during use or by using the aforementioned sealant.

In a similar manner, the covering layer 71 which corresponds to the backing of the wound dressing will be adapted to receive the connecting device of the vacuum system 1.

Such a modified absorbent wound dressing is, for example, used in the negative pressure wound treatment assembly exploited by the company SMITH & NEPHEW under the name PICO^{TM}_{.}

As can be seen on Figure 3, in addition to the vacuum system 1 and the covering layer 71, the negative pressure wound treatment assembly 70 comprises the above disclosed spacing 72 and wound contact 73 layers.

The spacing layer 72 is placed over the wound 80 with the wound contact layer 73 interposed between the spacing layer 72 and the wound 80. The wound contact layer 73 is for example URGOTUL® made by LABORATOIRES URGO. The spacing layer 72 can be an absorbing flexible layer, for example made of hydrophilic polyurethane foam. The covering layer 71 is adapted to form an airtight barrier covering the spacing layer 72 with a bottom surface intended to face the wound 80. The covering layer 71 can be a tight polyurethane film dressing OPTISKIN® made by LABORATOIRES URGO with an acrylic adhesive layer on the bottom surface to allow the covering layer 71 to be sealed around the wound 80.

After the wound contact layer 73 has been placed onto the wound 80 and the spacing layer 72 placed onto the wound contact layer 73, the covering layer 71 attached to the securing interface 60 of the vacuum system 1 is placed over the spacing layer 72 and sealed around the wound 80. The covering layer 71 thereby defines an air tight space surrounding the wound 80. In the represented embodiment, the securing interface 60 and its port 63 extends trough the covering layer 71 with the outer surface of the cup 61 in tight contact with the bottom surface of the covering layer 71, and the plug 65 arranged on a top surface of the covering layer 71. In doing so, the port 63 of the securing interface 60 opens in the bottom surface of the covering layer 71. In other embodiments, the port 63 of the securing interface 60 is not necessarily arranged through the covering layer 71. The port 63 could possibly be arranged at the edge of the covering layer 71 to open in the bottom surface of the covering layer, the securing interface 60 being adapted accordingly.

Then, as explained above, the holder 15 is connected to the securing interface 60 by inserting the plug 65 in the groove 33 of the distal end 18 of the body 16.

Once the port 63 is arranged coaxially with the body 16, the tube 2 can be inserted in the housing 21 of the body 16 through the proximal end 17 towards the needle portion 41 with its cover 44 in the closing position, the plunger 45 being in the retracted position. Thanks to the force exerted by the spring 50, the plunger 45 remains in the retracted position. The cap 6 of the tube 2 forces the cover 44 towards the tipped end which pierces the cover 44 and, once uncovered, perforates the cap 6. As the insertion movement of the tube 2 is continued, the cover 44 slides along the needle portion 41 to the opening position where it is collapsed close to the core 43 of the penetration member 40. The communication passage is partly vacuumized in the penetration member 40 but is tightly closed by the cover 44 of the connector 42.

A further downward action exerted on the tube 2 towards the distal end 18 of the holder 15 causes the plunger 45 to slide along the axis B of the body 16 towards the distal end 18. As the plunger 45 reaches the extended position, the connector 42 extends though the distal end 18 of the body 18 and the cover 44 in the closing position is in contact with the thick part 66 of the plug 65 of the securing interface 60. The tipped end of the connector 44 pierces the cover 44 and, once uncovered, enters the port 63 and perforates the membrane 68. As the downward action is continued, the cover 44 slides along the connector 42 to the opening position where it is collapsed close to the core 43 of the penetration member 40. In this position, the communication passage is fully opened and reduced pressure can be transferred to the inner surface 62 of the cup 61 and the bottom surface of the covering layer 71 through the port 63.

The plunger 45 may be maintained in the extended position for the determined duration by the locking arrangement. The tube 2 may be replaced by another, the removal of the tube 2 causing the cover 44 of the needle portion 41 to return back to the closed position. The plunger 45 can also be returned to the retracted position, the vacuum being prevented to escape by means of the membrane 68 of the securing interface 60. In this position, a further replacement of the tube 2 or a removal of the holder 15 from the securing interface 60 is possible.

The vacuum system 1 has been disclosed in relation to the first embodiment in which the connector 42 is formed of a needle portion on the moveable penetration member to be directly connected to the port 63 of the securing interface 60, the holder 15, especially the distal end 18 of its body 16, and the securing interface 60, especially the plug 65, being configured to be removably secured directly to each other.

The invention is however not limited to this first embodiment.

In particular, in other embodiments, the connector of the holder could be any kind of sleeve with a through bore, and not necessarily a tipped end, adapted to be mounted to the port of the securing interface. Besides, the connector could extend along an axis offset from the axis of the needle portion of the penetration member and could be arranged on a component of the holder other than the penetration member. For example, the connector could be arranged at the distal end of the body of the holder.

Besides, in other embodiments, the distal end of the holder and the securing interface could be otherwise connected.

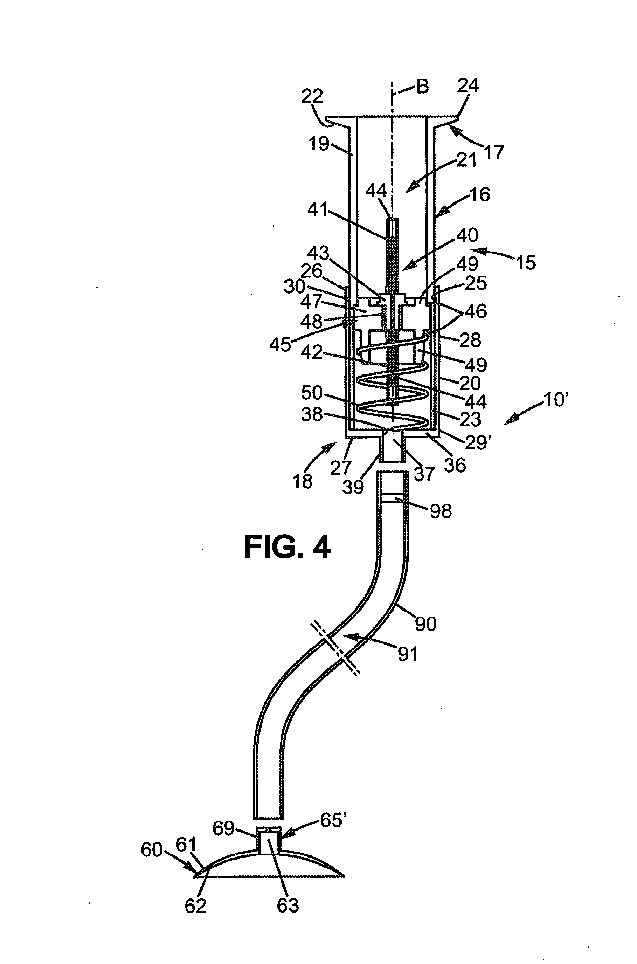

For example, Figure 4 represents a second embodiment of the vacuum system 1' in which the holder 15 and the securing interface 60 can be connected to each other while being spaced apart from each other, the connecting device 10' further comprising a flexible pipe 90 for connecting the holder 15 and the securing interface 60.

The vacuum device 1' of the second embodiment only differs from the vacuum device of the first embodiment in the way the holder 15 and the securing device 60 are connected to each other. For the elements that are common to the first and second embodiments, the same reference numbers will be used and the description will not be repeated. Reference is made to the description of these elements in relation to the first embodiment for more details.

As apparent from Figure 4, the body 16 of the holder 15 of the vacuum system 1' according to the second embodiment has the same structure as that of the vacuum system 1 according to the first embodiment except in the second end 27 of the holder cap 20 which forms the distal end 18 of the body 16. Actually, the second end 27 of the holder cap 20 has a transverse end portion 29' comprising a transverse wall 36 provided with a central opening 37 delimited by an internal edge 38, and an annular sleeve 39 extending along the axis B of the body 16, opposite to the housing 21, from the internal edge 38 of the transverse wall 36.

Besides, the securing interface 60 of the vacuum system 1' according to the second embodiment has the same structure as that of the vacuum system 1 according to the first embodiment except in the shape of the plug 65' secured to the cup 61. In The second embodiment, the plug 65' is formed of an annular sleeve 69 through which the port 63 extends.

The pipe 90 defines a conduit 91 extending along its whole length between two ends adapted to be mounted on the sleeve 39 of the holder 15 and the sleeve 69 of the securing interface 60, respectively. To enable the detachment of the holder 15 and the pipe 90 without loosing vacuum within the cavity of the cup 61, a membrane 98 acting as a valve member is provided at the end intended to be attached to the holder 15 to selectively close and open the conduit 91 respectively in closing and opening positions.

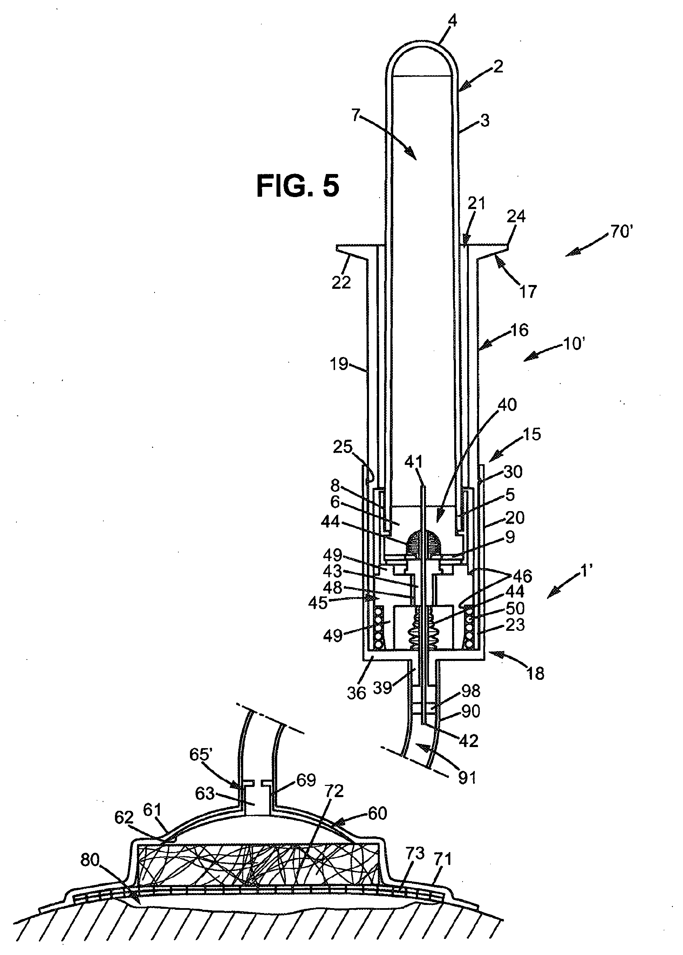

Figure 5 represents the vacuum system 1' of the second embodiment implemented in a reduced pressure wound treatment assembly 70' in which it is connected to the covering layer 71 to generate a reduced pressure on the wound 80 of a patient's body. This application of the vacuum system 1' of the second embodiment is very similar to that of the first embodiment and will not be disclosed in details.

The wound contact layer 73, the spacing layer 72, the covering layer 71 and the securing interface 60 are arranged in similar manner as previously disclosed with respect to the wound 80. The pipe 90 is fitted onto the sleeve 69 of the plug 65' of the securing interface 60' and onto the sleeve 39 of the holder 15.

As explained above, the tube 2 is inserted in the housing 21 of the body 16 until the cover 44 is collapsed close to the core 43 of the penetration member 40 to uncover the needle portion 41 and allow its penetration trough the cap 6. The communication passage within the needle portion 41 remains tightly closed by the cover 44 of the connector 42.

As the plunger 45 reaches the extended position with a further downward action exerted on the tube 2, the connector 42 reaches the distal end 18 of the body 18 and the cover 44 in the closing position is in contact with the transverse wall 36 of the holder 15. The tipped end of the connector 42 pierces the cover 44 and, once uncovered, extends through the sleeve 39, enters the pipe 90 and perforates the membrane 98. As the downward action is continued, the cover 44 slides along the connector 42 to the opening position where it is collapsed close to the core 43 of the penetration member 40. In this position, the communication passage is fully opened and reduced pressure can be transferred to the inner surface 62 of the cup 61 and the bottom surface of the covering layer 71 through the pipe 90 and the port 63.

## Claims

1. Negative pressure wound treatment assembly (70, 70') comprising:
- a covering layer (71) adapted to form an airtight barrier covering a wound (80), the covering layer (71) presenting a bottom surface intended to face the wound (80),
- a vacuum system (1, 1') adapted to be connected to the covering layer (71) to generate a reduced pressure on the wound (80) when said covering layer (71) covers said wound (80), wherein
the vacuum system (1,1') comprises:
- a chamber (2) having a tightly closed space (7) with a reduced pressure,
- a connecting device (10, 10') adapted to be attached to the covering layer (71) and to the chamber (2) to form a communication passage between the bottom surface of covering layer (71) and the space (7) of the chamber (2) the connecting device (10, 10') further comprising at least one valve member (44, 68, 98) moveable between a closing position, in which said valve member (44, 68, 98) closes the communication passage, and an opening position, in which said valve member (44, 68, 98) opens the communication passage,
**characterised in that** said chamber (2) comprises an end (5) closed by a perforable cap (6), and the connecting device (10, 10') has a penetration member (40) forming at least a part of the communication passage and has at least one needle portion (41, 42) adapted to perforate the cap (6) of the chamber (2) and further **in that** the valve member comprises a cover (44) mounted on the needle portion (41, 42) of the penetration member (40), the cover (44) being in the closing position in absence of external strain, said cover (44) covering a tipped end of said needle portion (41, 42) in the closing position, the cover (44) being adapted to be resiliently deformed to the opening position when an external strain is exerted so as to push the cover (44) in a direction opposite to the tipped end, said cover being perforated by the tipped end in the opening position.

2. Negative pressure wound treatment assembly (70, 70') according to claim 1, wherein the connecting device (10, 10') comprises:
- a securing interface (60) attached to the covering layer (71), said securing interface (60) comprising a port (63) opening in the bottom surface of the covering layer (71) and forming a part of the communication passage,
- a holder (15) having a housing (21) adapted to receive the chamber (2), the holder (15) comprising the penetration member (40) arranged within the housing (21) so as to perforate the cap (6) of the chamber (2) with the needle portion (41), the holder (15) comprising a connector (42) in communication with the needle portion (41) of the penetration member (40) and forming a part of the communication passage, the connector (42) being adapted to be mounted on the securing interface (60) in communication with the port (63).

3. Negative pressure wound treatment assembly (70, 70') according to claim 2, wherein the chamber is a tube (2) and the holder (15) comprises a hollow cylindrical body (16) extending along an axis (B) between opened proximal (17) and distal (18) ends and defining the housing (21), the penetration member (40) being mounted within the body (16) with the needle portion (41) directed towards the proximal end (17).

4. Negative pressure wound treatment assembly (70, 70') according to claim 3, wherein the distal end (18) of the body (16) is adapted to be removably secured to the securing interface (60).

5. Negative pressure wound treatment assembly (70, 70') according to any of claims 2 to 4, wherein the holder (15) comprises a plunger (45) supporting the needle portion (41) of the penetration member (40) and the connector (42), said plunger (45) being mounted within the housing (21) so as to slide between a retracted position, in which the connector (42) is fully contained in the housing (21), and an extended position, in which the connector (42) at least partly protrudes outwardly from the housing (21).

6. Negative pressure wound treatment assembly (70, 70') according to claim 5, wherein the plunger (45) is resiliently returned to the retracted position.

7. Negative pressure wound treatment assembly (70, 70') according to claim 6, wherein the holder (15) comprises a spring (50) arranged within the housing (21) and interposed between a distal end (18) of the holder (15) and the plunger (45), the spring (50) being sized to make it possible to perforate the cap (6) of the chamber (2) by the needle portion (41) while the plunger (45) remains in the retracted position and then to move the plunger (45) towards the extended position upon a further action on the chamber (2) towards the distal end (18) of the holder (15) after the cap (6) has been perforated.

8. Negative pressure wound treatment assembly (70, 70') according to any of claims 2 to 7, wherein the connector (42) is adapted to be fitted within the port (63) of the securing interface (60).

9. Negative pressure wound treatment assembly (70, 70') according to any of claims 2 to 8, wherein the connector (42) is a portion of the penetration member (40) extending in a direction which is opposite to that of the needle portion (41).

10. Negative pressure wound treatment assembly (70, 70') according to any of claims 2 to 9, wherein the securing interface (60) comprises a cup (61) having a concave inner surface (62) defining a cavity intended to face the wound (80), the port (63) opening within the cavity.

11. Negative pressure wound treatment assembly (70, 70') according to any of claims 2 to 10, wherein the securing interface (60) comprises a plug (65, 65') through which the port (63) extends, the plug (65, 65') being adapted to be removably secured to the holder (15).

12. Negative pressure wound treatment assembly (70, 70') according to claim 2 to 11, wherein the connecting device further comprises a flexible pipe (90) removably secured to the securing interface (60) and to the holder (15), said pipe (90) delimiting a conduit (91) forming a part of the communication passage.

13. Negative pressure wound treatment assembly (70, 70') according to any of claims 2 to 12, wherein the connector (42) is a needle portion and the valve member comprises a cover (44) mounted on the connector (42), the cover (44) being in the closing position in absence of external strain, said cover (44) covering a tipped end of said connector (42) in the closing position, the cover (44) being adapted to be resiliently deformed to the opening position when an external strain is exerted so as to push the cover (44) in a direction opposite to the tipped end, said cover being perforated by the tipped end in the opening position.

14. Negative pressure wound treatment assembly (70, 70') according to any of claims 2 to 13, wherein the valve member comprises a membrane (68) on the securing interface (60), the membrane (68) closing and opening the port (63) respectively in the closing and opening positions.

15. Negative pressure wound treatment assembly (70, 70') according to claim 12, wherein the valve member comprises a membrane (98) within the pipe (90), the membrane (98) closing and opening the conduit (91) respectively in the closing and opening positions.

## Patentansprüche

1. Negativdruck-Wundenbehandlungs-Baugruppe (70, 70'), umfassend:
- eine Abdeckschicht (71), welche dazu eingerichtet ist, eine luftdichte Barriere zu bilden, welche eine Wunde (80) abdeckt, wobei die Abdeckschicht (71) eine untere Oberfläche darstellt, welche dazu vorgesehen ist, zu der Wunde (80) zu weisen,
- ein Vakuum-System (1, 1'), welches dazu eingerichtet ist, mit der Abdeckschicht (71) verbunden zu werden, um einen reduzierten Druck an der Wunde (80) zu erzeugen, wenn die Abdeckschicht (71) die Wunde (80) abdeckt, wobei
das Vakuum-System (1, 1') umfasst:
- eine Kammer (2), welche einen dicht abgeschlossenen Raum (7) mit einem reduzierten Druck aufweist,
eine Verbindungs-Vorrichtung (10, 10'), welche dazu eingerichtet ist, an die Abdeckschicht (71) und an die Kammer (2) angebracht zu werden, um einen Verbindungs-Durchgang zwischen der unteren Oberfläche der Abdeckschicht (71) und dem Raum (7) der Kammer (2) zu bilden, wobei die Verbindungs-Vorrichtung (10, 10') ferner wenigstens ein Ventil-Element (44, 68, 98) umfasst, welches zwischen einer Schließ-Position, in welcher das Ventil-Element (44, 68, 98) den Verbindungs-Durchgang verschließt, und einer Öffnungs-Position, in welcher das Ventil-Element (44, 68, 98) den Verbindungs-Durchgang öffnet, bewegbar ist,
**dadurch gekennzeichnet, dass** die Kammer (2) ein von einer durchstechbaren Kappe (6) verschlossenes Ende (5) umfasst und die Verbindungs-Vorrichtung (10, 10') ein Durchdringungs-Element (40) aufweist, welches wenigstens einen Teil des Verbindungs-Durchgangs bildet und wenigstens einen Nadelabschnitt (41, 42) aufweist, welcher dazu eingerichtet ist, die Kappe (6) der Kammer (2) zu perforieren,
und ferner dadurch, dass
das Ventil-Element eine Abdeckung (44) umfasst, welche an dem Nadelabschnitt (41, 42) des Durchdringungs-Elements (40) montiert ist, wobei die Abdeckung (44) sich in der Abwesenheit von externer Beanspruchung in der Schließ-Position befindet, wobei die Abdeckung (44) ein angespitztes Ende des Nadelabschnitts (41, 42) in der Schließ-Position abdeckt, wobei die Abdeckung (44) dazu eingerichtet ist, elastisch zu der Öffnungs-Position verformt zu werden, wenn eine externe Beanspruchung ausgeübt wird, so dass die Abdeckung (44) in eine Richtung entgegengesetzt dem angespitzten Ende geschoben wird, wobei die Abdeckung von dem angespitzten Ende in der Öffnungs-Position perforiert wird.

2. Negativdruck-Wundenbehandlungs-Baugruppe (70, 70') nach Anspruch 1, wobei die Verbindungs-Vorrichtung (10, 10') umfasst:
- eine an der Abdeckschicht (71) angebrachte Sicherungs-Schnittstelle (60), wobei die Sicherungs-Schnittstelle (60) einen Anschluss (63) umfasst, welcher sich in der unteren Oberfläche der Abdeckschicht (71) öffnet und einen Teil des Verbindungs-Durchgangs bildet,
- ein ein Gehäuse (21) aufweisendes Halteelement (15), welches dazu eingerichtet ist, die Kammer (2) aufzunehmen, wobei das Halteelement (15) das Durchdringungs-Element (40) umfasst, welches innerhalb des Gehäuses (21) derart angeordnet ist, dass es die Kappe (6) der Kammer (2) mit dem Nadelabschnitt (41) perforiert, wobei das Halteelement (15) ein Verbindungselement (42) in Verbindung mit dem Nadelabschnitt (41) des Durchdringungs-Elements (40) umfasst und einen Teil des Verbindungs-Durchgangs bildet, wobei das Verbindungselement (42) dazu eingerichtet ist, an der Sicherungs-Schnittstelle (60) in Verbindung mit dem Anschluss (63) montiert zu werden.

3. Negativdruck-Wundenbehandlungs-Baugruppe (70, 70') nach Anspruch 2, wobei die Kammer eine Röhre (2) ist, und das Halteelement (15) einen hohlen zylindrischen Körper (16) umfasst, welcher sich entlang einer Achse (B) zwischen geöffneten proximalen (17) und distalen (18) Enden erstreckt und das Gehäuse (21) definiert, wobei das Durchdringungs-Element (40) innerhalb des Körpers (16) montiert ist, wobei der Nadelabschnitt (41) in Richtung des proximalen Endes (17) gerichtet ist.

4. Negativdruck-Wundenbehandlungs-Baugruppe (70, 70') nach Anspruch 3, wobei das distale Ende (18) des Körpers (16) dazu eingerichtet ist, entfernbar an der Sicherungs-Schnittstelle (60) gesichert zu sein.

5. Negativdruck-Wundenbehandlungs-Baugruppe (70, 70') nach einem der Ansprüche 2 bis 4, wobei das Halteelement (15) einen Stößel (45) umfasst, welcher den Nadelabschnitt (41) des Durchdringungs-Elements (40) und das Verbindungselement (42) trägt, wobei der Stößel (45) innerhalb des Gehäuses (21) montiert ist, um zwischen einer zurückgezogenen Position, in welcher das Verbindungselement (42) vollständig in dem Gehäuse (21) enthalten ist, und einer ausgefahrenen Position, in welcher das Verbindungselement (42) wenigstens teilweise nach außen aus dem Gehäuse (21) hervorsteht, zu gleiten.

6. Negativdruck-Wundenbehandlungs-Baugruppe (70, 70') nach Anspruch 5, wobei der Stößel (45) elastisch in die zurückgezogene Position zurückgeführt wird.

7. Negativdruck-Wundenbehandlungs-Baugruppe (70, 70') nach Anspruch 6, wobei das Halteelement (15) eine Feder (50) umfasst, welche innerhalb des Gehäuses (21) angeordnet und zwischen einem distalen Ende (18) des Halteelements (15) und dem Stößel (45) eingefügt ist, wobei die Feder (50) derart bemessen ist, dass sie es ermöglicht, die Kappe (6) der Kammer (2) mittels des Nadelabschnitts (41) zu perforieren, während der Stößel (45) in der zurückgezogenen Position verbleibt, und dann auf ein weiteres Einwirken auf die Kammer (2) in Richtung des distalen Endes (18) des Halteelements (15) hin, nachdem die Kappe (6) perforiert worden ist, den Stößel (45) in Richtung der ausgefahrenen Position zu bewegen.

8. Negativdruck-Wundenbehandlungs-Baugruppe (70, 70') nach einem der Ansprüche 2 bis 7, wobei das Verbindungselement (42) dazu eingerichtet ist, innerhalb des Anschlusses (63) der Sicherungs-Schnittstelle (60) eingepasst zu werden.

9. Negativdruck-Wundenbehandlungs-Baugruppe (70, 70') nach einem der Ansprüche 2 bis 8, wobei das Verbindungselement (42) ein Abschnitt des Durchdringungs-Elements (40) ist, welcher sich in einer Richtung erstreckt, welche derjenigen des Nadelabschnitts (41) entgegengesetzt ist.

10. Negativdruck-Wundenbehandlungs-Baugruppe (70, 70') nach einem der Ansprüche 2 bis 9, wobei die Sicherungs-Schnittstelle (60) eine Schale (61) umfasst, welche eine konkave innere Oberfläche (62) aufweist, welche einen Hohlraum definiert, welcher dazu vorgesehen ist, zu der Wunde (80) zu weisen, wobei sich der Anschluss (63) innerhalb des Hohlraums öffnet.

11. Negativdruck-Wundenbehandlungs-Baugruppe (70, 70') nach einem der Ansprüche 2 bis 10, wobei die Sicherungs-Schnittstelle (60) einen Stecker (65, 65') umfasst, durch welchen sich der Anschluss (63) erstreckt, wobei der Stecker (65, 65') dazu eingerichtet ist, entfernbar an dem Halteelement (15) gesichert zu sein.

12. Negativdruck-Wundenbehandlungs-Baugruppe (70, 70') nach Anspruch 2 bis 11, wobei die Verbindungs-Vorrichtung ferner ein flexibles Rohr (90) umfasst, welches entfernbar an der Sicherungs-Schnittstelle (60) und an dem Halteelement (15) gesichert ist, wobei das Rohr (90) eine Leitung (91) begrenzt, welche einen Teil des Verbindungs-Durchgangs bildet.

13. Negativdruck-Wundenbehandlungs-Baugruppe (70, 70') nach einem der Ansprüche 2 bis 12, wobei das Verbindungselement (42) ein Nadelabschnitt ist und das Ventil-Element eine Abdeckung (44) umfasst, welche an dem Verbindungselement (42) montiert ist, wobei die Abdeckung (44) sich in der Abwesenheit von externer Beanspruchung in der Schließ-Position befindet, wobei die Abdeckung (44) ein angespitztes Ende des Verbindungselements (42) in der Schließ-Position abdeckt, wobei die Abdeckung (44) dazu eingerichtet ist, elastisch zu der Öffnungs-Position verformt zu werden, wenn eine externe Beanspruchung ausgeübt wird, so dass die Abdeckung (44) in eine Richtung entgegengesetzt dem angespitzten Ende geschoben wird, wobei die Abdeckung von dem angespitzten Ende in der Öffnungs-Position perforiert wird.

14. Negativdruck-Wundenbehandlungs-Baugruppe (70, 70') nach einem der Ansprüche 2 bis 13, wobei das Ventil-Element eine Membran (68) an der Sicherungs-Schnittstelle (60) umfasst, wobei die Membran (68) den Anschluss (63) jeweils in die Schließ- und Öffnungs-Position schließt und öffnet.

15. Negativdruck-Wundenbehandlungs-Baugruppe (70, 70') nach Anspruch 12, wobei das Ventil-Element eine Membran (98) innerhalb des Rohrs (90) umfasst, wobei die Membran (98) die Leitung (91) jeweils in die Schließ- und Öffnungs-Position schließt und öffnet.

## Revendications

1. Ensemble de traitement de plaies par pression négative (70, 70') comprenant :
- une couche de recouvrement (71) conçue pour former une barrière étanche à l'air couvrant une plaie (80), la couche de recouvrement (71) présentant une surface inférieure destinée à être orientée vers la plaie (80),
- un système d'aspiration (1, 1') conçu pour être raccordé à la couche de recouvrement (71) pour générer une pression réduite sur la plaie (80) lorsque ladite couche de recouvrement (71) couvre ladite plaie (80), dans lequel le système d'aspiration (1, 1') comprend :
- une chambre (2) ayant un espace fermé hermétiquement (7) avec une pression réduite,
- un dispositif de raccordement (10, 10") conçu pour être attaché à la couche de recouvrement (71) et à la chambre (2) pour former un passage de communication entre la surface inférieure de la couche de recouvrement (71) et l'espace (7) de la chambre (2), le dispositif de raccordement (10, 10') comprenant en outre au moins un élément formant valve (44, 68, 98) mobile entre une position de fermeture, dans laquelle ledit élément formant valve (44, 68, 98) ferme le passage de communication, et une position d'ouverture, dans laquelle ledit élément formant valve (44, 68, 98) ouvre le passage de communication,
**caractérisé en ce que** ladite chambre (2) comprend une extrémité (5) fermée par un capuchon perforable (6), et le dispositif de raccordement (10, 10') possède un élément de pénétration (40) constituant au moins une partie du passage de communication et ayant au moins une partie formant aiguille (41, 42) conçue pour perforer le capuchon (6) de la chambre (2), et **en ce que**, en outre, l'élément formant valve comprend un étui (44) monté sur la partie formant aiguille (41, 42) de l'élément de pénétration (40), l'étui (44) étant dans la position de fermeture en l'absence de contrainte externe, ledit étui (44) couvrant une extrémité en pointe de ladite partie formant aiguille (41, 42) dans la position de fermeture, l'étui (44) étant conçu pour être déformé de manière élastique vers la position d'ouverture lorsqu'une contrainte externe est exercée, afin de pousser l'étui (44) dans une direction opposée à l'extrémité en pointe, ledit étui étant perforé par l'extrémité en pointe dans la position d'ouverture.

2. Ensemble de traitement de plaies par pression négative (70, 70') selon la revendication 1, dans lequel le dispositif de raccordement (10, 10') comprend :
- une interface de fixation (60) attachée à la couche de recouvrement (71), ladite interface de fixation (60) comprenant un orifice (63) s'ouvrant dans la surface inférieure de la couche de recouvrement (71) et faisant partie du passage de communication,
- un support (15) ayant un logement (21) conçu pour recevoir la chambre (2), le support (15) comprenant l'élément de pénétration (40) disposé à l'intérieur du logement (21) afin de perforer le capuchon (6) de la chambre (2) avec la partie formant aiguille (41), le support (15) comprenant un connecteur (42) en communication avec la partie formant aiguille (41) de l'élément de pénétration (40) et faisant partie du passage de communication, le connecteur (42) étant conçu pour être monté sur l'interface de fixation (60) en communication avec l'orifice (63).

3. Ensemble de traitement de plaies par pression négative (70, 70') selon la revendication 2, dans lequel la chambre est un tube (2) et le support (15) comprend un corps cylindrique creux (16) s'étendant le long d'un axe (B) entre les extrémités proximale (17) et distale (18) ouvertes et définissant le logement (21), l'élément de pénétration (40) étant monté à l'intérieur du corps (16) avec la partie formant aiguille (41) dirigée vers l'extrémité proximale (17).

4. Ensemble de traitement de plaies par pression négative (70, 70') selon la revendication 3, dans lequel l'extrémité distale (18) du corps (16) est conçue pour être fixée de manière amovible à l'interface de fixation (60).

5. Ensemble de traitement de plaies par pression négative (70, 70') selon l'une quelconque des revendications 2 à 4, dans lequel le support (15) comprend un piston (45) supportant la partie formant aiguille (41) de l'élément de pénétration (40) et le connecteur (42), ledit piston (45) étant monté à l'intérieur du logement (21) de manière à coulisser entre une position rétractée, dans laquelle le connecteur (42) est complètement contenu dans le logement (21), et une position déployée, dans laquelle le connecteur (42) fait saillie au moins partiellement vers l'extérieur du logement (21).

6. Ensemble de traitement de plaies par pression négative (70, 70') selon la revendication 5, dans lequel le piston (45) est retourné de manière élastique à la position rétractée.

7. Ensemble de traitement de plaies par pression négative (70, 70') selon la revendication 6, dans lequel le support (15) comprend un ressort (50) disposé à l'intérieur du logement (21) et intercalé entre une extrémité distale (18) du support (15) et le piston (45), le ressort (50) étant de taille à permettre la perforation du capuchon (6) de la chambre (2) par la partie formant aiguille (41) alors que le piston (45) reste dans la position rétractée et ensuite, le déplacement du piston (45) vers la position déployée lors d'une autre action sur la chambre (2) vers l'extrémité distale (18) du support (15) une fois que le capuchon (6) a été perforé.

8. Ensemble de traitement de plaies par pression négative (70, 70') selon l'une quelconque des revendications 2 à 7, dans lequel le connecteur (42) est conçu pour être installé dans l'orifice (63) de l'interface de fixation (60).

9. Ensemble de traitement de plaies par pression négative (70, 70') selon l'une quelconque des revendications 2 à 8, dans lequel le connecteur (42) est une partie de l'élément de pénétration (40) s'étendant dans une direction qui est opposée à celle de la partie formant aiguille (41).

10. Ensemble de traitement de plaies par pression négative (70, 70') selon l'une quelconque des revendications 2 à 9, dans lequel l'interface de fixation (60) comprend une coupelle (61) ayant une surface interne concave (62) définissant une cavité censée être orientée vers la plaie (80), l'orifice (63) s'ouvrant dans la cavité.

11. Ensemble de traitement de plaies par pression négative (70, 70') selon l'une quelconque des revendications 2 à 10, dans lequel l'interface de fixation (60) comprend un connecteur (65, 65') à travers lequel s'étend l'orifice (63), le connecteur (65, 65') étant conçu pour être fixé de manière amovible au support (15).

12. Ensemble de traitement de plaies par pression négative (70, 70') selon les revendications 2 à 11, dans lequel le dispositif de raccordement comprend en outre une tuyauterie souple (90) fixée de manière amovible à l'interface de fixation (60) et au support (15), ladite tuyauterie (90) délimitant un conduit (91) faisant partie du passage de communication.

13. Ensemble de traitement de plaies par pression négative (70, 70') selon l'une quelconque des revendications 2 à 12, dans lequel le connecteur (42) est une partie formant aiguille et l'élément formant valve comprend un étui (44) monté sur le connecteur (42), l'étui (44) étant dans la position de fermeture en l'absence de contrainte externe, ledit étui (44) couvrant une extrémité en pointe dudit connecteur (42) dans la position de fermeture, l'étui (44) étant conçu pour être déformé de manière élastique vers la position ouverte lorsqu'une contrainte externe est exercée afin de pousser l'étui (44) dans une direction opposée à l'extrémité en pointe, ledit étui étant perforé par l'extrémité en pointe dans la position ouverte.

14. Ensemble de traitement de plaies par pression négative (70, 70') selon l'une quelconque des revendications 2 à 13, dans lequel l'élément formant valve comprend une membrane (68) sur l'interface de fixation (60), la membrane (68) fermant et ouvrant l'orifice (63) respectivement dans les positions de fermeture et d'ouverture.

15. Ensemble de traitement de plaies par pression négative (70, 70') selon la revendication 12, dans lequel l'élément formant valve comprend une membrane (98) à l'intérieur de la tuyauterie (90), la membrane (98) fermant et ouvrant le conduit (91) respectivement dans les positions de fermeture et d'ouverture.
